# EUROPÄISCHE PATENTSCHRIFT

(11) **EP 0 554 570 B1**
(45) Veröffentlichungstag und Bekanntmachung des Hinweises auf die Patenterteilung: **13.10.2004**
(21) Anmeldenummer: 92122126.3
(22) Anmeldetag: 30.12.1992
(51) Int. Cl.: A61K 35/14, A61K 38/36

(54) **Verfahren zur Herstellung einer stabilen Fibrinogenlösung**
Procedure for the manufacture of a stable fibrinogen solution
Procédé de production d'une solution stable de fibrinogène

(30) Priorität: 29.01.1992 DE 4202667
(43) Veröffentlichungstag der Anmeldung: 11.08.1993
(60) Teilanmeldung: 03012650.2
(73) Patentinhaber: ZLB Behring GmbH, 35041 Marburg (DE)
(72) Erfinder: Hock, Johann, W-3550 Marburg (DE); Karges, Hermann E., W-3550 Marburg-Michelbach (DE)

(56) Entgegenhaltungen:
- EP-A- 0 014 333
- EP-A- 0 103 196
- EP-A- 0 305 243
- EP-A- 0 311 950

## Beschreibung

Die Erfindung betrifft ein Verfahren zur Herstellung einer stabilen Fibrinogenlösung, die für die Verwendung in einem Gewebekleber geeignet ist und mindestens vier Wochen ohne Verlust ihrer Funktionsfähigkeit, insbesondere ohne wesentliche Veränderung der Konsistenz und der Gerinnungseigenschaften bei Raumtemperatur gelagert werden kann.

Die Gewebeklebung und Blutstillung mit Fibrin wird seit langem durchgeführt. Sie wird beispielsweise eingesetzt zur Stillung großflächiger Blutungen, zur Gewebevereinigung in parenchymatösen Organen, zur Bindehautklebung oder zur Nahtsicherung und Blutstillung nach verschiedenen chirurgischen Eingriffen. Der wesentliche Vorteil gegenüber anderen Methoden (z. B. Klebung mit synthetischen Gewebeklebern) ist die Verwendung von körpereigenen Substanzen, die eine gute Verträglichkeit aufweisen und die Wundheilung positiv beeinflussen. Bei der Gewebeklebung wird die letzte Phase der Blutgerinnung nachvollzogen: Das Fibrinogen, welches Faktor XIII enthält, wird auf der zu klebenden Fläche mit Thrombin und Calciumionen in Kontakt gebracht. Das Thrombin spaltet aus dem Fibrinogen die Fibrinopeptide ab, wobei Fibrinmonomere entstehen, die ein lösliches Fibringerinnsel bilden. Gleichzeitig wird durch Thrombin und Calciumionen die Transglutaminase Faktor XIII aktiviert, die das Gerinnsel durch Quervernetzung der Fibrinmonomere stabilisiert. Eine erhöhte Resistenz des Gerinnsels gegenüber Fibrinolyse kann, falls gewünscht, durch Zusatz eines Plasmin-Inhibitors zu dem Fibrinogenkonzentrat erreicht werden.

Für die Gewebeklebung mit Fibrinklebern stehen einerseits kommerzielle Präparate zur Verfügung, andererseits können auch Fibrinkleber bei Bedarf durch den Anwender hergestellt werden (WO 86/01814).

Die kommerziell erhältlichen Fibrinkleber sind Zweikomponentenkleber, wobei die erste Komponente Fibrinogen, Faktor XIII und Aprotinin, die zweite Thrombin und Calciumionen enthält. Die Komponenten liegen entweder tiefgefroren oder lyophilisiert vor. Ein Nachteil dieser Präparate ist die erforderliche Vorbereitung des gebrauchsfertigen Klebers, da sowohl das Auftauen und Erwärmen auf Anwendungstemperatur, als auch das Auflösen der Lyophilisate - bedingt durch die hohe Fibrinogenkonzentration von ca. 6-10% - relativ viel Zeit beanspruchen. Ein weiterer wesentlicher Nachteil ist die relativ kurze Haltbarkeit (ca. 4 Stunden) der gebrauchsfertigen Kleberkomponenten. Bei unvorhergesehenen Verzögerungen zwischen Vorbereitung und Anwendung kann so der Fibrinkleber unbrauchbar werden oder an Wirksamkeit verlieren.

Fibrinogenkonzentrate können auch nach verschiedenen Verfahren bei Bedarf durch den Anwender selbst hergestellt und mit kommerziell erhältlichen Thrombinpräparaten zu einem Gewebekleber kombiniert werden. Dieses Vorgehen ist jedoch mit folgenden gravierenden Nachteilen behaftet: Um die Übertragung von Viren auszuschließen, kann nur Blut des zu behandelnden Patienten für die Gewinnung des Fibrinogenkonzentrats verwendet werden. Da die Herstellung des Konzentrates arbeits- und zeitaufwendig ist, ist die Anwendung in Notfällen nicht möglich. Weiterhin ist bei Verwendung von Blut eines einzelnen Spenders eine gleichbleibende Zusammensetzung und Qualität des Fibrinogenkonzentrats nicht gewährleistet.

In der Europäischen Patentanmeldung 0 014 333 A1 werden unter anderem Verfahren beschrieben, in denen Fibrinogen an kolloidales Silikagel (Aeorosil®) gebunden und davon eluiert wird.

Aufgrund der angeführten Nachteile sowohl der kommerziellen als auch der vom Anwender selbst hergestellten Fibrinogenkonzentrate besteht ein Bedarf an einem Fibrinogenkonzentrat, das in der gebrauchsfertigen Form (aufgetaut bzw. gelöst) eine möglichst lange Haltbarkeit aufweist. Das erfindungsgemäße Fibrinogenkonzentrat behebt beide Nachteile der bisherigen Gewebekleber. Ein Vorteil hierbei ist eine schnellere Verfügbarkeit, da die zum Auftauen bzw. Lösen der Fibrinogenkomponente benötigte Zeit entfällt. Ein weiterer Vorteil ist der, dass die gelöste Fibrinogenkomponente über einen längeren Zeitraum funktionsfähig bleibt und nicht verworfen werden muss, wenn eine vorgesehene Klebung nicht durchgeführt wird.

Aufgabe der Erfindung war daher die Bereitstellung eines Verfahrens zur Herstellung eines Fibrinogenkonzentrats, das mindestens 4 Wochen ohne Verlust der Funktionsfähigkeit bei + 4°C bis + 25°C lagerbar ist. Eine solche Lösung soll besonders in einem Gewebekleber verwendbar sein.

EP 0 085 923 beschreibt die Herstellung eines argininhaltigen Fibriongenkonzentrats, das über einen Arbeitstag stabil bleibt.

Überraschenderweise wurde nun ein Verfahren zur Herstellung einer Fibinogenlösung gefunden, die über einen längeren Zeitraum gelagert werden kann, ohne wesentlich an Gerinnbarkeit und damit ihrer Eignung für eine Gewebeklebung zu verlieren. Das Verfahren ist dadurch gekennzeichnet, dass eine Fibinogenlösung mindestens zwei Adsoptionsschritten unterworfen wird.

Eine auf diese Weise behandelte Lösung erlaubt auch nach Auftauen aus dem tiefgefrorenen Zustand oder nach Lösen aus dem lyophilisierten Zustand eine Lagerung über einen längeren Zeitraum, ohne ihre Funktionsfähigkeit zu verlieren.

Gegenstand der Erfindung ist deshalb ein Verfahren zur Herstellung einer konzentrierten Fibrinogenlösungmit 1,5 % bis 15 % gerinnbarem Plasmaprotein, die mindestens vier Wochen bei +4 bis +25° C gelagert werden kann, ohne ihre Funktionsfähigkeit zu verlieren, wobei ein fibrinogenhaltiges Ausgangsmaterial, welches gerinnbares Plasmaprotein enthält, mindestens zweimal mit einem Adsorptionsmittel behandelt wird, wobei das Adsorptionsmittel Al(OH)₃, ein Anionenaustauscher oder das schwerlösliche Salz eines Erdalkalimetalls ist und jeweils der Überstand gewonnen wird.

Als Fibrinogenlösung kann eine Lösung eines Kryopräzipitates verwendet werden. Eine solche Lösung kann 1,5 - 15, vorzugsweise 5 - 11% gerinnbares Plasmaprotein enthalten.

Die Konzentration des Erdalkalisalzes oder Aluminiumhydroxids bei der Adsorption liegt zwischen 0.05 und 2.5 % (Gew.Nol.), vorzugsweise 0.05 - 0.5 % (Gew./Vol.) und die verwendete Menge an Ionenaustauscher ist 0.05 - 20 g vorzugsweise 3 - 10 g pro kg Kryopräzipitat.

Die Fibrinogenlösung kann gegebenenfalls nach dem Fachmann bekannten Verfahren (z. B. EP 0 103 196) weiter gereinigt werden. Die so gewonnene Fibrinogenlösung kann flüssig gelagert, tiefgefroren oder lyophilisiert werden. Die Lagerung der Fibinogenlösung oder einer nach Einfrieren und Auftauen der Lösung oder Auflösen einer getrockneten Lösung erhaltenen Fibrinogenlösung kann bei einer Temperatur von + 1°C bis + 37°C erfolgen. Bevorzugt wird bei einer Temperatur von + 4°C bis + 25°C gelagert. Eine erfindungsgemäße Lösung ist für mindestens 4 Wochen stabil.

Eine nach dem erfindungsgemäßen Verfahren hergestellte stabile Lösung kann als Komponente eines Fibrinklebers verwendet werden.

Die folgenden Beispiele erläutern die Erfindung:

### Beispiel 1: Herstellung eines Fibrinogenkonzentrats

Kryopräzipitat aus Citratplasma wurde in isotonischer Kochsalzlösung (2.8 l/kg) gelöst. Anschließend wurden 5% (Vol./Vol.) einer Aluminiumhydroxid-Suspension (1.5% Gew./Vol.) zugesetzt, und die Mischung 15 Min. gerührt. Das Aluminiumhydroxid wurde abzentrifugiert, dem überstand wurde nochmals die gleiche Menge Aluminiumhydroxid sowie 5.25 g QAE-Sephadex A-50 pro kg Kryopräzipitat zugesetzt. Nach 15 Min. wurde erneut zentrifugiert. Dem Überstand wurde Glycin bis zu einer Endkonzentration von 2.7 mol/l unter Rühren zugesetzt, der entstehende Niederschlag abzentrifugiert und in isotonischer Kochsalzlösung (1.5 l/kg) gelöst. Die Lösung wurde mit Glycin versetzt (Endkonzentration 1.15 mol/l) und 30 Min. gerührt. Danach wurde zentrifugiert und der Rückstand verworfen. Der Überstand wurde mit Glycin (Endkonzentration 2.15 mol/l) versetzt und 30 Min. gerührt. Der Niederschlag wurde abzentrifugiert, in 0.05 mol/l NaCl, 0.005 mol/l tri-Natrium-Citrat, 0.02 mol/l Arginin, pH 7.5, aufgenommen und gegen denselben Puffer dialysiert.

### Beispiel 2: Herstellung eines Faktor XIII enthaltenden Fibrinogenkonzentrats

Das Fibrinogenkonzentrat nach Beispiel 1 wurde mit 20 E/ml Faktor XIII aus Plasma und 3.3 mg/ml Humanalbumin versetzt. Die Lösung wurde auf eine optische Dichte (280 nm) von 35-37 eingestellt und lyophilisiert.

### Beispiel 3: Stabilität von Faktor XIII enthaltenden Fibrinogenkonzentraten in Lösung bei +4-8°C

Lyophilisierte Fibrinogenkonzentrate wurden mit 1 ml einer Lösung von 1000 KIU/ml Aprotinin in physiologischer Kochsalzlösung gelöst und bei +4-8°C gelagert. Die Konzentration des gerinnbaren Fibrinogens wurde mit dem Test nach Clauss bestimmt, die Aktivität von Faktor XIII mit einem chromogenen Test (^{R}Berichrom Faktor XIII).

| A | | | B | |
|---|---|---|---|---|
| **Tage** | **Fgn**^{**1)**} | **FXIII**^{**2)**} | **Fgn** | **F XIII** |
| 0 | 100 | 64 | 101 | 10.3 |
| 2 | 101 | 55 | 61 | 9.2 |
| 6 | 109 | 59 | fest³⁾ | fest |
| 15 | 108 | 61 | | |
| 18 | 86 | 56 | | |
| 26 | 91 | 61 | | |
| 30 | 88 | 58 | | |
| A) Fibrinogenkonzentrat nach Beispiel 1 mit plasmatischem Faktor XIII B) nicht adsorbiertes Kryopräzipitat als Fibrinogenkonzentrat | | | | |

| | | | | |
|---|---|---|---|---|
| 1) Fibrinogen (mg/ml) | | | | |
| 2) Faktor XIII (E/ml) | | | | |
| 3) wird bei Erwärmen nicht mehr flüssig | | | | |

### Beispiel 4: Stabilität von Faktor XIII enthaltendem Fibrinogenkonzentrat in Lösung bei +20°C

Ein erfindungsgemäßes, lyophilisiertes Fibrinogenkonzentrat wurde in 1 ml einer Lösung von 1000 KIU/ml Aprotinin in physiologischer Kochsalzlösung gelöst und bei +20°C gelagert. Die Konzentration des gerinnbaren Fibrinogens wurde mit dem Test nach Clauss bestimmt, die Aktivität von Faktor XIII mit einem chromogenen Test (^{R}Berichrom Faktor XIII).

| **Tage** | **Fgn**^{**1)**} | **FXIII**^{**2)**} |
|---|---|---|
| 0 | 80 | 65 |
| 2 | 87 | 65 |
| 4 | 89 | 66 |
| 9 | 86 | 63 |
| 18 | 88 | 62 |
| 22 | 82 | 71 |
| 32 | 84 | 61 |

| | | |
|---|---|---|
| 1) Fibrinogen (mg/ml) | | |
| 2) Faktor XIII (E/ml) | | |

## Patentansprüche

1. Verfahren zur Herstellung einer konzentrierten Fibrinogenlösung mit 1,5 % bis 15 % gerinnbarem Plasmaprotein, die mindestens vier Wochen bei + 4 bis + 25°C gelagert werden kann, ohne ihre Funktionsfähigkeit zu verlieren, **dadurch gekennzeichnet, dass** fibrinogenhaltiges Ausgangsmaterial, welches gerinnbares Plasmaprotein enthält, mindestens zweimal mit einem Adsorptionsmittel behandelt wird, wobei das Adsorptionsmittel Al(OH)₃, ein Anionenaustauscher oder das schwerlösliche Salz eines Erdalkalimetalls ist und jeweils der Überstand gewonnen wird.

2. Verfahren nach Anspruch 1, **dadurch gekennzeichnet, dass** als geeignete Ausgangslösung Kryopräzipitat genommen wird.

3. Verfahren nach Anspruch 1 und 2, **dadurch gekennzeichnet, dass** zweimal mit Al(OH)₃ und einmal mit QAE-Sephadex® A-50 behandelt wird.

## Claims

1. A process for preparing a concentrated fibrinogen solution which contains 1.5% to 15% of coagulable plasma protein and which can be stored at +4 to +25°C for at least four weeks without losing its ability to function, which comprises treating a fibrinogen-containing starting material, which contains coagulable plasma protein, at least twice with an adsorbent, the adsorbent being Al(OH)₃, an anion exchanger or the sparingly soluble salt of an alkaline earth metal, and obtaining the supernatant each time.

2. The process as claimed in claim 1, wherein cryoprecipitate is taken as suitable starting solution.

3. The process as claimed in claims 1 and 2, wherein there is treatment twice with Al(OH)₃ and once with QAE-Sephadex® A-50.

## Revendications

1. Procédé de préparation d'une solution fibrinogène concentrée avec 1,5 % à 15 % de protéine plasmique coagulable, qui peut être stockée pendant au moins 4 semaines entre +4°C et +25°C, sans perdre sa capacité fonctionnelle, **caractérisé en ce que** la matière de départ contenant le fibrinogène, qui comprend la protéine plasmique coagulable, est traitée au moins deux fois avec un milieu d'absorption, ledit milieu d'absorption étant Al(OH)₃, un échangeur d'anions, ou un sel difficilement soluble d'un métal alcalino-terreux, et **en ce qu'**on récupère le cas échéant le milieu résultant.

2. Procédé selon la revendication 1, **caractérisé en ce que** l'on utilise un cryoprécipité à titre de solution de départ appropriée.

3. Procédé selon la revendication 1 et 2, **caractérisé en ce que** l'on effectue le traitement deux fois avec Al(OH)₃ et une fois avec du QAE-Sephadex® A-50.
